(19) Europäisches Patentamt
European Patent Office
Office européen des brevets

(11) Numéro de publication: **0 301 977 B1**

(12) # FASCICULE DE BREVET EUROPEEN

(45) Date de publication de fascicule du brevet: **11.11.92**

(51) Int. Cl.5: **C08H 1/06**, A61L 27/00

(21) Numéro de dépôt: **88420240.9**

(22) Date de dépôt: **08.07.88**

(54) **Procédé de réticulation du collagène par introduction de groupes azides ainsi que les tissus et biomatériaux obtenus par mise en oeuvre du procédé.**

(30) Priorité: **08.07.87 FR 8710317**

(43) Date de publication de la demande:
**01.02.89 Bulletin 89/05**

(45) Mention de la délivrance du brevet:
**11.11.92 Bulletin 92/46**

(84) Etats contractants désignés:
**AT BE CH DE ES FR GB GR IT LI LU NL SE**

(56) Documents cités:

**BIOTECHNOLOGY AND BIOENGINEERING, vol. XXVI, 1984, pages 665-669, John Wiley & Sons Inc., R. EL HABIB et al.: "DNA immobilized onto an acyl-azide derivative of collagen membranes for use as immunoadsorbent"**

**CHEMICAL ABSTRACTS, vol. 75, 1971, page 135, résumé no. 127858q, Columbus, Ohio, US; C.I. LEVENE: "Effect of lathyrogenic compounds on the crosslinking of collagen and elastin in vivo", & SYMP. MECH. TOXICITY 1970 (PUB. 1971), 67-85**

(73) Titulaire: **COLETICA**
**32, rue Saint Jean de Dieu**
**F-69007 Lyon(FR)**

Titulaire: **INSTITUT NATIONAL DE LA SANTE ET DE LA RECHERCHE MEDICALE (INSERM)**
**101, rue de Tolbiac**
**F-75654 Paris Cédex 13(FR)**

(72) Inventeur: **Petite, Hervé**
**118 avenue Saint-Exupéry**
**F-69500 Bron(FR)**
Inventeur: **Menasche, Philippe**
**1 rue du Regard**
**F-75006 Paris(FR)**
Inventeur: **Huc, Alain**
**26 Chemin des Santons**
**F-69110 Sainte Foy Les Lyon(FR)**

(74) Mandataire: **Maureau, Pierre et al**
**Cabinet GERMAIN & MAUREAU B.P. 3011**
**F-69392 Lyon Cédex 03(FR)**

EP 0 301 977 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

HOPPE-SEYLER'S ZEITSCHRIFT FÜR PHYSIOLOGISCHE CHEMIE, vol. 356, september 1975, pages 1455-1458, Walter de Gruyter, Berlin, DE; W.F. TROMMER et al.: "Synthesis and properties of a new selective bifunctional cross-linking reagent"

CHEMICAL ABSTRACTS, vol. 92, 1980, page 70, résumé no. 165227s, Columbus, Ohio, US; J.M. ADZET et al.: "Hydrothermal stability of bated hides treated with various salts", & AOEIC BOL. TEC. 1979, 30(11), 301-15

## Description

La présente invention concerne un procédé de réticulation du collagène par introduction de groupes azides ainsi que les tissus et biomatériaux obtenus par mise en oeuvre du procédé.

Le remplacement d'une valve cardiaque pathologique chez l'homme fait actuellement essentiellement appel à deux types de substituts : les prothèses mécaniques et les valves biologiques hétérologues (encore dénommés hétérogreffes ou bioprothèses). Si les premières ont l'avantage d'une durabilité presque illimitée, elles ont comme limitation principale un risque thrombo-embolique permanent obligeant à un traitement anticoagulant à vie dont les risques propres (hémorragiques) contribuent pour une part non négligeable à la morbidité et à la mortalité globale observées chez les patients porteurs de ces prothèses.

A l'opposé les bioprothèses ont aujourd'hui clairement fait la preuve de leur faible thrombogénicité, et ce en l'absence de traitement anticoagulant. En revanche, leur longévité est fortement obérée par la survenue de calcifications tissulaires qui, isolées ou en association avec des lésions dites de fatigue, conduisent à une réintervention dans un délai moyen de 6 ans, ce délai étant d'ailleurs souvent plus court chez les malades les plus jeunes.

Aussi, l'un des principaux courants de recherche en matière de valves artificielles est-il aujourd'hui axé sur la mise au point de techniques permettant de retarder la détérioration des bioprothèses.

Cette détérioration est, à l'évidence, un phénomène plurifactoriel, certains facteurs dits "liés à l'hôte" étant difficilement contrôlables.

Il apparaît toutefois clairement que le mode de traitement du tissu, au moment de la fabrication de la valve, traitement sur lequel il est possible d'intervenir, peut jouer un rôle primordial.

A l'heure actuelle, quelle que soit l'origine animale de ce tissu (valves aortiques de porc ou péricarde bovin), son traitement fait pratiquement toujours appel,pour les modèles de bioprothèses actuellement sur le marché, à des techniques de tannage utilisant le glutaraldéhyde (G.T.A.).

On connaît en effet, l'aptitude de cet agent tannant à créer des liaisons intermoléculaires solides qui permettront au tissu ainsi traité de présenter les propriétés mécaniques requises et de résister à la dégradation enzymatique "in vivo".

Un argument supplémentaire pour l'utilisation du glutaraldéhyde est que, par création de liaisons entre les molécules de collagène, il masque certains déterminants antigéniques ce qui permet de limiter l'antigénicité du tissu.

Bien que la filiation exacte entre le glutaraldéhyde et la calcification ne soit pas encore totalement élucidée, il existe certainement une relation de causalité. Il apparait en effet que, contrairement aux implants non traités, les implants de valves aortiques de porc traités au G.T.A. se calcifient sévèrement.

De plus, on a démontré que des valves aortiques de porc, ou du péricarde de veau, tous deux traités au GTA, calcifient de manière similaire chez le rat.(Schoen et al Am.J. Pathol 1986, 193 pp 134-145).

Bien que des facteurs régulant ce mécanisme de calcification et dépendant de l'hôte et de l'implant aient été mis en évidence (par Lévy, Schoen, Howard et al), on n'a pas encore pu déterminer le mécanisme sous-jacent de cette calcification.

Le rôle des groupements latéraux du collagène a par ailleurs été étudié par Urist en 1966 et Glimcher en 1968.

Ainsi Urist a pu montrer que le traitement de tendon de queue de rat dans des solutions de sels inorganiques telles que CaCl2, SrCl2 ou des cations organiques, qui bloquent les groupements carboxyliques des chaînes latérales, inhibe efficacement la calcification des tendons implantés chez le rat hypercalcémique.

Glimcher a tenté d'inhiber la calcification du collagène de l'os in vitro en estérifiant les groupements carboxyliques. Dans ce cas, le blocage des groupements inhibe la calcification. Cependant, une partie de cette inhibition serait due à la déshydratation de la molécule dans le méthanol.

La présente invention s'est donné pour but de proposer un procédé chimique susceptible d'assurer le bloquage des groupements latéraux acides du collagène et d'éviter l'utilisation de glutaraldéhyde dont l'inconvénient est en plus de l'induction de calcification, une certaine toxicité.

Dans FR-A-2 559 666 & EP-A-156 740 les inventeurs avaient déjà proposé un procédé de fabrication de tubes de collagène selon lequel, une fois séché, le tube de collagène était soumis à une réticulation par déshydratation poussée en étuve à environ 80° C sous un vide d'environ 0,1 mm de mercure pendant environ 24 heures.

Le tube de collagène réticulé était ensuite soumis à un traitement permettant d'introduire des groupes azides dans la molécule sans toutefois occasionner le couplage du collagène avec quelque molécule que ce soit.

Les inventeurs ont poursuivi leurs recherches dans cette voie notamment en essayant d'éliminer l'étape

préalable de réticulation par déshydratation tout en arrivant à un bloquage des groupements terminaux acides du collagène améliorant les propriétés mécaniques du tissu obtenu et évitant toute resolubilisation du collagène, le but étant d'obtenir un degré de réticulation du collagène équivalent à celui obtenu avec le glutaraldéhyde laissant ainsi espérer une diminution des phénomènes de calcification et bien entendu des phénomènes de toxicité liés au relargage secondaire du glutaraldéhyde.

L'intérêt du procédé est renforcé par le fait qu'il ne remplace pas le glutaraldéhyde par un autre agent tannant qui pourrait poser des problèmes spécifiques, puisqu'il crée des liaisons réticulaires entre les chaînes latérales du collagène sans introduire de façon permanente un agent chimique étranger.

Ainsi, sur le plan conceptuel, le procédé selon l'invention parait-il présenter une voie nouvelle dans la mesure où il substitue à la fixation traditionnelle du tissu, invariablement fondée sur l'emploi du glutaraldéhyde, une fixation fondée sur une modification de structure des groupements latéraux du collagène obtenue sans recours à un quelconque agent tannant.

Les inventeurs ont pu déterminer au cours de leur recherche qu'il était possible de bloquer efficacement les groupements latéraux du collagène de façon à éviter toute resolubilisation de ce dernier et d'améliorer de façon importante les propriétés mécaniques des biomatériaux obtenus si l'on effectue une réticulation chimique du collagène par introduction de groupes azides en présence de chlorure de sodium.

Selon l'invention, le procédé de traitement d'un collagène non réticulé par introduction de groupes azides qui comporte essentiellement les étapes suivantes :

a) estérification des groupements acides libres du collagène.

b) transformation des groupements estérifiés en groupements hydrazides

c) transformation des groupements hydrazides en groupements azides par action de l'acide nitreux,

est caractérisé en ce que en vue d'une réticulation dudit collagène non réticulé, on fait réagir, au cours d'une étape d), les groupements azide obtenus à l'étape c), avec les groupements aminés du même collagène et entre les étapes a) et b) et/ou entre les étapes b) et c), on rince le collagène traité avec une solution aqueuse de sel, ce sel n'ayant aucune action dénaturante sur le collagène, et les étapes a, b et c sont effectuées en présence de sel non dénaturant du collagène.

Le sel est choisi parmi les sels de métaux alcalins ne dénaturant pas le collagène. Les solutions aqueuses de sel sont utilisées à des concentrations se situant entre 3 et 15 %. La réaction de réticulation par introduction de groupes azides est effectuée sur les collagènes non réticulés.

L'invention concerne également les biomatériaux obtenus par mise en oeuvre du procédé ci-avant et notamment les bio-prothèses ainsi que tout autre biomatériau à base du collagène réticulé ainsi obtenu, tels que films, éponges, tubes, etc.

La présente invention sera mieux comprise et ses avantages ressortiront bien de la description qui suit d'un mode de réalisation du procédé selon l'invention.

A Préparation du matériau de départ

On prélève aux abattoirs, les péricardes, chez des veaux de 100 jours, moins d'une heure après leur mise à mort. Ces péricardes sont alors dégraissés, triés, lavés dans une solution aqueuse à 9 ‰ de chlorure de sodium.

B Réticulation du collagène par introduction de groupes azides

Cette réaction de réticulation peut être résumée comme suit :

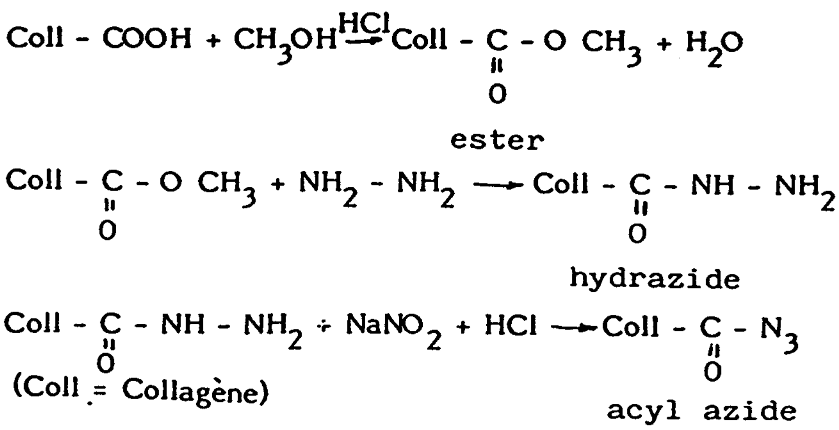


La première étape a), l'estérification des groupements carboxyliques est réalisée par immersion des échantillons dans une solution de méthanol contenant 0,2 mole d'acide chlorhydrique à température ambiante pendant 7 jours.

Deux rinçages sont effectués dans le méthanol sans acide.

Puis on procède à deux rinçages avec une solution aqueuse à 6 % de chlorure de sodium (étape a').

Les échantillons sont ensuite plongés dans une solution d'hydrazine 1 % avec NaCl 6 % pendant une nuit afin d'obtenir des hydrazides à partir des groupements estérifiés. La réaction se passe à température ambiante (étape b).

On procède alors à un rinçage à 4° C avec une solution aqueuse à 6 % de chlorure de sodium (étape b').

L'étape suivante (étape c), consiste à immerger les échantillons obtenus dans une solution renfermant 0,3 mole d'acide chlorydrique et 0,5 mole de nitrite de sodium dans une solution aqueuse à 6 % de chlorure de sodium.

Les échantillons sont ensuite rincés (étape c') afin d'en éliminer complètement l'acide ; ils sont ensuite maintenus pendant quatre heures à température ambiante dans un tampon borate-chlorure de sodium 6 %.

Les acyl-azotures formés à partir des groupements carboxyliques des chaînes latérales du collagène vont ainsi pouvoir réagir au cours de l'étape d), avec les groupements aminés, essentiellement de la lysine, pour former une liaison amide. On crée ainsi des liaisons de réticulation à l'intérieur du réseau collagénique.

Les échantillons sont enfin incubés, à température ambiante, dans un tampon borate-glycine-chlorure de sodium à 9 % pendant au moins trois heures.

Afin d'obtenir un biomatériau stérile, il est préférable de conserver les échantillons ainsi obtenus dans de l'alcool à 70°.

L'influence exercée par l'addition du chlorure de sodium au cours des différentes étapes du procédé va maintenant être étudiée.

Dans le dessin schématique annexé :

Figures 1 et 2 sont des courbes de calorimétrie différentielle programmées respectivement pour un péricarde frais non traité, et pour un péricarde traité selon l'invention.

Figure 3 et 4 sont des courbes retraçant les propriétés physiques (force de rupture en N en abscisse, et allongement en mm en ordonnée) respectivement pour un tendon frais et pour un tendon traité selon l'invention.

Figure 5 est une courbe retraçant l'évolution du gonflement d'une peau en fonction du pH.

1) Etude en calorimétrie différentielle programmée

L'analyse calorimétrique différentielle programmée (C.D.P.) permet une étude calorimétrique dynamique des échantillons expérimentaux.

La technique consiste à mesurer, lors d'une montée linéaire de la température, la différence d'énergie qu'il faut fournir à un échantillon et à une référence pour les maintenir à une température identique. Lorsque la protéine se dénature, un pic d'absorption de chaleur apparaît sur l'enregistreur. En effet, la transition hélice pelote statique est endothermique pour le collagène.

On définit ainsi :

- la température de début de dénaturation

- la température de pic de dénaturation
- la température de fin de dénaturation.

L'étude a été effectuée comparativement :

- sur un film et sur un péricarde témoin, non traité (témoin)
- sur un film et sur un péricarde soumis aux différentes étapes de réticulation par introduction de groupes azides, en présence d'eau.
- sur un film et sur un péricarde soumis aux différentes étapes de réticulation par introduction de groupes azides selon l'invention c'est-à-dire en présence d'une solution aqueuse à 6 % de chlorure de sodium (NaCl).
- sur un film et sur un péricarde traité avec une solution à 0,6 % de glutaraldéhyde (G.T.A.).

Les résultats obtenus sont rassemblés dans le tableau I ci-après :

Tableau I

| | Temps en °C Début | Temps en °C Pic | Temps en °C Fin |
|---|---|---|---|
| Film | | | |
| témoin | 39,15 | 49,1 | 66,1 |
| eau | 53,6 | 63,8 | 76,6 |
| NaCl 6 % | 65,5 | 74,3 | 83 |
| GTA 0,6 % | 71,1 | 79,1 | 86,4 |
| Péricarde | | | |
| témoin | 53,6 | 62,8 | 73,6 |
| eau | 70 | 75,7 | 84,1 |
| NaCl 6 % | 78 | 81,4 | 89,1 |
| GTA 0,6 % | 82 | 85,3 | 93,8 |

La figure 1 représente la courbe énergie/température pour un péricarde frais et la figure 2 la même courbe pour un péricarde traité par le procédé selon l'invention, dans un milieu ne contenant pas de chlorure de sodium aux étapes n° a', b, b', c, c', ce péricarde a une température de début de dénaturation de 70° C en fin de traitement.

Un péricarde traité dans les mêmes conditions mais en présence de NaCl 6 % aux étapes n° a', b, b', c, c', selon l'invention, a une température de début de dénaturation de 78° C en fin de traitement.

Des résultats similaires sont relevés sur les films où l'on observe, dans le cas de films traités par le procédé selon l'invention, une augmentation de température de début de dénaturation de 12° C.

2) Digestion à la collagénase

Afin de confirmer les résultats obtenus en calorimétrie différentielle et d'évaluer les performances des matériaux face à des "agressions" se rapprochant de celles que l'on peut retrouver in-vivo, les différents matériaux (traité en l'absence de sel, traité en présence de chlorure de sodium, traité au G.T.A.) ont été incubés pendant 7 jours en présence de collagénase bactérienne (600 U/ml).

Le 7ème jour, on dose la quantité de protéines se retrouvant dans le surnageant. La quantité de protéines est proportionnelle au degré d'attaque par la collagénase.

Les résultats sont rassemblés dans le tableau II ci-après :

Tableau II

| Traitement | Taux de protéines |
|---|---|
| Traité en l'absence de NaCl | 105 µg/ml |
| Traité en présence de NaCl 6 % | 18 µg/ml |
| Traité G.T.A. 0,6 % | 6 µg/ml |

On constate également une tenue du matériau très nettement améliorée quand le traitement a été effectué en présence de chlorure de sodium.

3)Etude des propriétés mécaniques

L'influence des conditions du procédé de réticulation chimique sur les propriétés physiques du collagène a été étudiée sur des tendons de porc. Contrairement au péricarde, le tendon comporte des fibres de collagène toutes orientées dans la même direction ce qui facilite l'interprétation des résultats.

Les résultats sont rassemblés dans le tableau III et dans les figures 3 et 4.

La figure 3 est la courbe de la force de rupture en N en fonction de l'allongement en mm pour un tendon frais et la figure 4 est une courbe similaire pour un tendon traité par le procédé selon l'invention.

Lorsque le tendon est étiré, on obtient une courbe à partir de laquelle on peut déterminer :

- la force de rupture : elle est donnée par la force minimale nécessaire pour rompre l'échantillon
- l'allongement à la rupture exprimé en pourcentage de la longueur initiale de l'échantillon
- Le module d'élasticité : c'est la pente de la partie linéaire de la courbe ; plus le module d'élasticité est fort, plus le tissu est rigide.

Tableau III

| Etude des propriétés physiques en traction uniaxiale | | | |
|---|---|---|---|
| | force de rupture en N | allongement de rupture en % | Module élasticité en MPa |
| Tendon frais | 455,4 ± 88,3 | 1,88 ± 0,6 | 0,135 |
| Tendon activé par l'eau | 861,6 ± 269,7 | 4,4 ± 0,9 | 0,76 ± 0,32 |
| Tendon activé NaCl 6 % | 856,7 ± 171,7 | 5,9 ± 1,2 | 0,85 ± 0,38 |
| Tendon GTA 0,6 % | 924,9 ± 162,6 | 7,14 ± 1,9 | 0,78 ± 0,18 |

Les forces de rupture des tissus traités sont similaires et significativement différentes de la force de rupture d'un tissu témoin non traité. On constate, de plus, une augmentation des valeurs de l'allongement de rupture pour les tissus traités selon l'invention et tannés au G.T.A..

Enfin, on observe une augmentation significative du module d'élasticité qui traduit une augmentation de la rigidité.

Comment peut-on expliquer les différences importantes exposées ci-avant, notamment entre les matériaux soumis de façon classique, à une réticulation par introduction de groupes azides et les matériaux traités selon l'invention, en présence de chlorure de sodium ?

Quand l'introduction de groupes azides est effectuée en l'absence de chlorure de sodium, l'acide chlorhydrique entraîne en fin d'estérification, un gonflement du tissu ; celui-ci devient alors plus épais et translucide. Ce gonflement correspond à une dissolution limitée du tissu puisqu'il existe de nombreuses liaisons inter et intramoléculaires empêchant une dissolution totale du tissu. En fin d'estérification, la stabilité thermique du tissu est celle d'un collagène acido-soluble (la température de début de dénaturation est de 36° C, l'épaisseur du péricarde passe de 0,38 mm à 0,82 mm).

Aux autres étapes du traitement,le phénomène est identique mais de moindre amplitude.

Quand l'introduction de groupes azides se fait dans les conditions du procédé de l'invention, c'est-à-dire en présence de chlorure de sodium, on obtient, en fin d'estérification, un tissu qui, d'un point de vue macroscopique, ne s'est pas modifié.

En CDP, la stabilité thermique du tissu ainsi traité est voisine de celle d'un tissu frais non traité (la température de début de dénaturation est de 53,9° C pour le tissu traité selon l'invention et de 58° C pour le tissu frais).

Au cours des autres étapes de traitement, on n'observe pas non plus de dénaturation du tissu.

En théorie, on peut expliquer comme suit l'action limitante du chlorure de sodium sur le gonflement.

Le collagnène possède des groupements latéraux polaires provenant des acides aminés acides et basiques.

Ainsi, suivant le pH du milieu, ces groupements seront ou non ionisés.

En milieu acide : COOH et $NH_3^+$
En milieu basique : $COO^-$ et $NH_2$

Ces ionisations expliquent les phénomènes de gonflement c'est-à-dire d'absoption d'eau par la fibre.

Ils sont bien connus en tannerie et sont utilisés pour faciliter l'épilage des peaux. On a pu ainsi établir des courbes de gonflement de la peau en fonction du pH (figure 5).

L'estérification réalisée en présence d'acide chlorhydrique provoque la fixation d'ions H+ par le tissu. Ainsi, il va y avoir une migration d'H+ et de Cl- de la phase aqueuse à la phase collagénique. Afin de

maintenir l'électroneutralité, les ions chlorure et hydrogène circuleront sous forme de molécules plutôt que d'ions libres. Les ions hydrogène sont fixés par la protéine et la concentration en ions chlorure de la fibre se trouve être plus importante que la concentration en ions chlorure de la phase aqueuse. En accord avec la loi d'action de masse, le produit de la concentration en ions chlorure par la concentration en ions hydrogène doit être égal dans la fibre et dans la phase aqueuse. Il va alors se développer une pression osmotique dans la fibre qui va augmenter de volume en absorbant de l'eau d'où un gonflement du péricarde.

Le même raisonnement peut être tenu en milieu basique, lorsque le tissu se trouve en présence d'hydrazine (pH ≃ 10).

En présence de sel, on assiste à une répression du gonflement du tissu. En effet, dans ce cas la différence de concentration en ions chlorure est atténuée par adjonction de chlorure de sodium. La pression osmotique est faible et le gonflement réduit.

On constate, après estérification en présence de sel, une constance de l'épaisseur du tissu, une augmentation de la stabilité thermique ; une concentration en chlorure de sodium de 6 % s'est révélée être tout spécialement efficace pour obtenir un tannage important et de bonne qualité.

Pour résumer, il apparait que la réticulation du collagène par introduction de groupes azides en présence de chlorure de sodium, permet d'obtenir un matériau réticulé de meilleure qualité que le même traitement en l'absence de sel.

Le matériau réticulé en présence de chlorure de sodium présente un certain nombre de propriétés (stabilité thermique, digestion à la collagénase) qui sont voisines de celles d'un matériau traité au glutaraidéhyde.

Par rapport à un matériau traité au glutaraldéhyde, le matériau traité par le procédé selon l'invention, présente une biocompatibilité nettement améliorée.

En effet, le procédé selon l'invention présente le grand avantage de n'introduire aucun agent chimique étranger dans le collagène, alors que le traitement au glutaraldéhyde entraîne la fixation de substances qui sont cytotoxiques et qui, de ce fait, font perdre au tissu l'essentiel de ses propriétés biologiques.

De plus, un tissu traité par le procédé selon l'invention et implanté dans un organisme vivant ne relarguera aucune matière toxique pouvant exercer des effets fâcheux, à court ou à long terme.

Comme il va de soi, et comme il ressort de ce qui précède, le procédé qui vient d'être décrit, appliqué au péricarde peut être appliqué à tout autre tissu collagénique. Il peut bien entendu convenir à la réalisation de tout biomatériau à base de collagène reconstitué tels que films, éponges, tubes etc.

## Revendications

**1.** Procédé de traitement d'un collagène non réticulé par introduction de groupes azide qui comporte essentiellement les étapes suivantes :
   a) estérification des groupements acides libres du collagène
   b) transformation des groupements estérifiés en groupements hydrazide
   c) transformation des groupements hydrazide en groupements azide par action de l'acide nitreux

   caractérisé en ce que en vue d'une réticulation dudit collagène non réticulé, on fait réagir, au cours d'une étape d), les groupements azide obtenus à l'étape c), avec les groupements aminés du même collagène et entre les étapes a) et b) et/ou entre les étapes b) et c), on rince le collagène traité avec une solution aqueuse de sel, ce sel n'ayant aucune action dénaturante sur le collagène, et les étapes a), b) et c) sont effectuées en présence de sel non dénaturant du collagène.

**2.** Procédé selon la revendication 1, caractérisé en ce que le sel est choisi parmi les sels de métaux alcalins.

**3.** Procédé selon la revendication 2, caractérisé en ce que la concentration de la solution aqueuse de sel se situe entre 3 et 15 %.

**4.** Application du procédé selon l'une quelconque des revendications 1 à 3 à l'obtention de bioprothèses.

**5.** Application du procédé selon l'une quelconque des revendications 1 à 4 à l'obtention de biomatériaux.

## Claims

**1.** Process for treating a noncrosslinked collagen by the introduction of azide groups, which essentially

comprises the following steps:

a) esterification of the free acid groups of the collagen,

b) conversion of the esterified groups to hydrazide groups,

c) conversion of the hydrazide groups to azide groups by the action of nitrous acid,

characterised in that in order to crosslink the said noncrosslinked collagen, the azide groups obtained in step c) are reacted, during step d), with the amino groups of the same collagen and the treated collagen is rinsed, between steps a) and b) and/or between steps b) and c), with an aqueous solution of salt, this salt not having any denaturing action on the collagen, and steps a, b and c are performed in the presence of a salt which is nondenaturing for collagen.

**2.** Process according to Claim 1, characterised in that the salt is chosen from alkali metal salts.

**3.** Process according to Claim 2, characterised in that the concentration of aqueous salt solution is between 3 and 15%.

**4.** Application of the process according to any one of Claims 1 to 3, to the production of bioprostheses.

**5.** Application of the process according to any one of Claims 1 to 4, to the production of biomaterials.

## Patentansprüche

**1.** Verfahren zur Behandlung eines nicht vernetzten Kollagens durch Einführung von Azidgruppen, das im wesentlichen die folgenden Schritte umfaßt:

a) Verestern der freien Säuregruppierungen des Kollagens

b) Transformation der veresterten Gruppierungen in Hydrazidgruppierungen

c) Transformation der Hydrazidgruppierungen in Azidgruppierungen durch die Wirkung von salpetriger Säure

dadurch gekennzeichnet, daß man im Hinblick auf eine Vernetzung des nicht vernetzten Kollagens in einem Schritt d) die in dem Schritt c) erhaltenen Azidgruppierungen mit den Aminogruppierungen desselben Kollagens reagieren läßt, und daß man zwischen den Schritten a) und b) und/oder zwischen den Schritten b) und c) das behandelte Kollagen mit einer wässrigen Salzlösung wäscht, wobei dieses Salz keinerlei denaturierenden Einfluß auf das Kollagen hat, und daß die Schritte a), b) und c) in Anwesenheit eines das Kollagen nicht denaturierenden Salzes durchgeführt werden.

**2.** Verfahren nach Anspruch 1, dadurch gekennzeichnet, daß das Salz aus den Salzen der Alkalimetalle ausgewählt wird.

**3.** Verfahren nach Anspruch 2, dadurch gekennzeichnet, daß die Konzentration der wässrigen Salzlösung zwischen 3 und 15 % liegt.

**4.** Anwendung des Verfahrens nach einem der Ansprüche 1 bis 3 zum Erhalten von Bioprothesen.

**5.** Anwendung des Verfahrens nach einem der Ansprüche 1 bis 4 zum Erhalten von Biomaterialien.

FIG.1

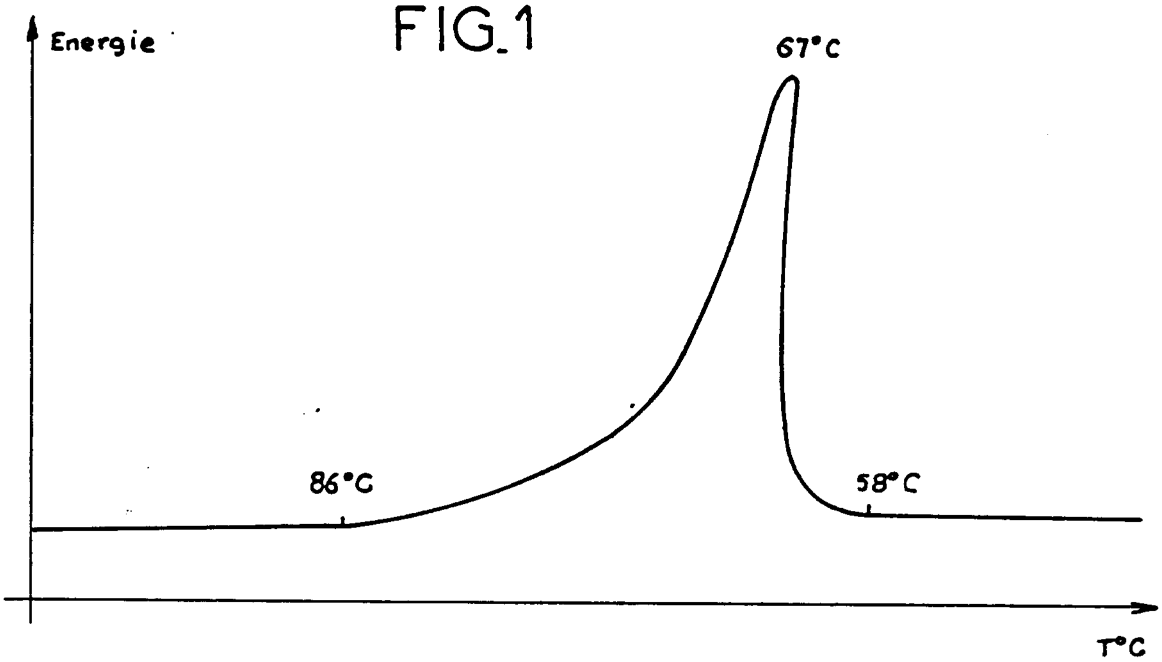


FIG.2

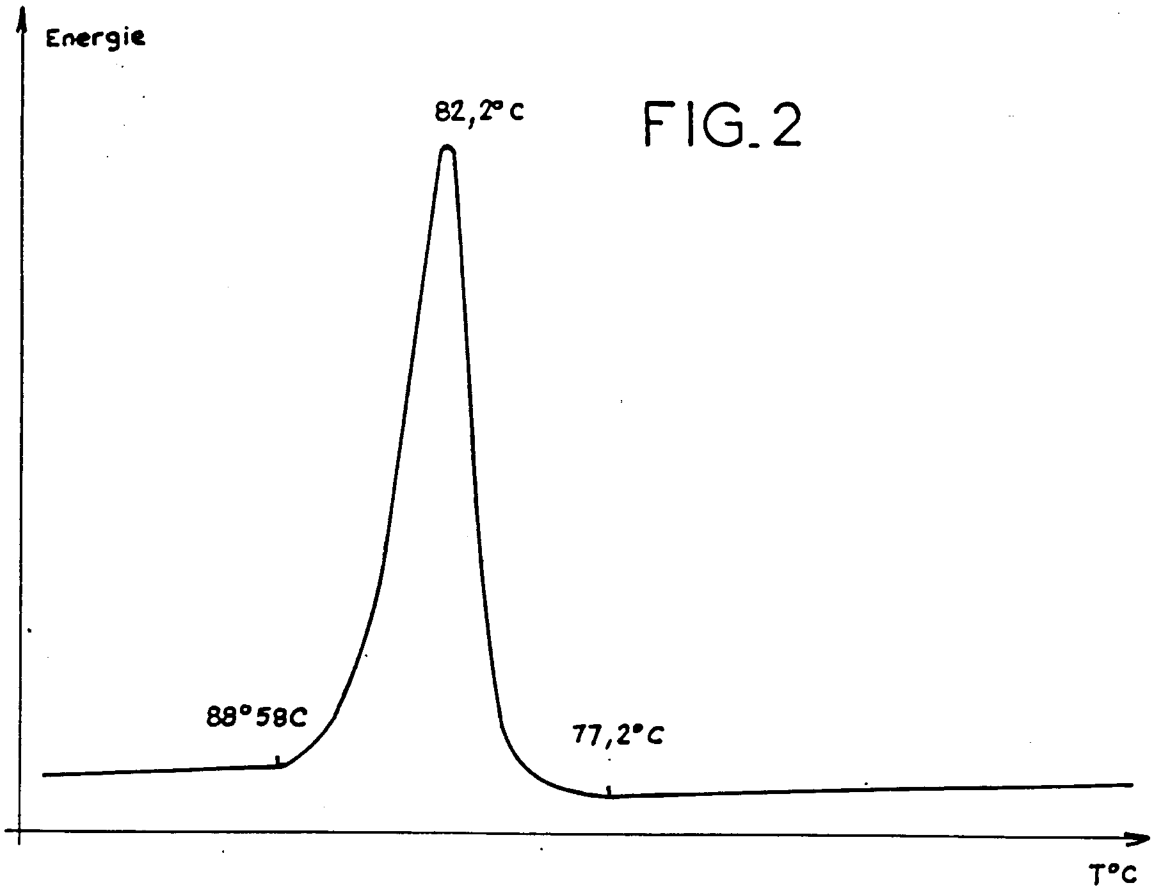

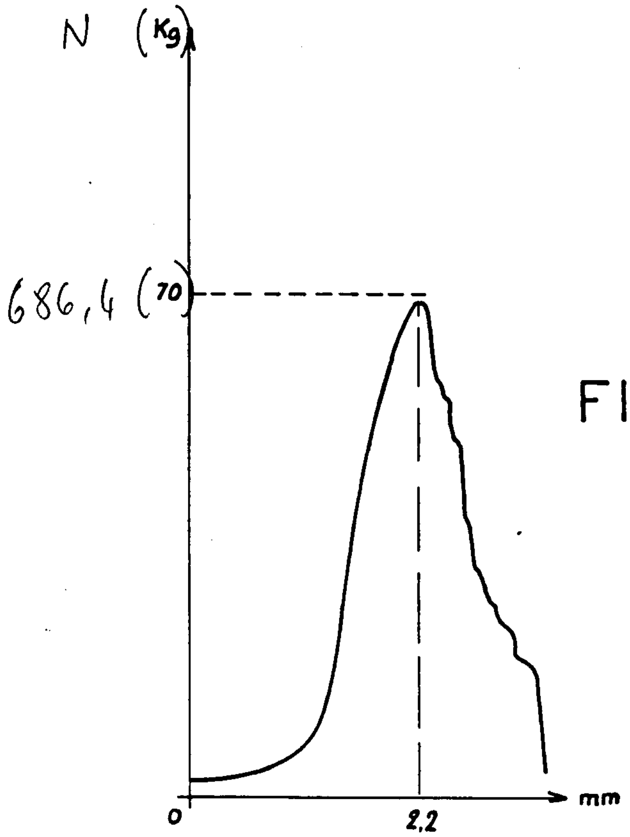


FIG.3

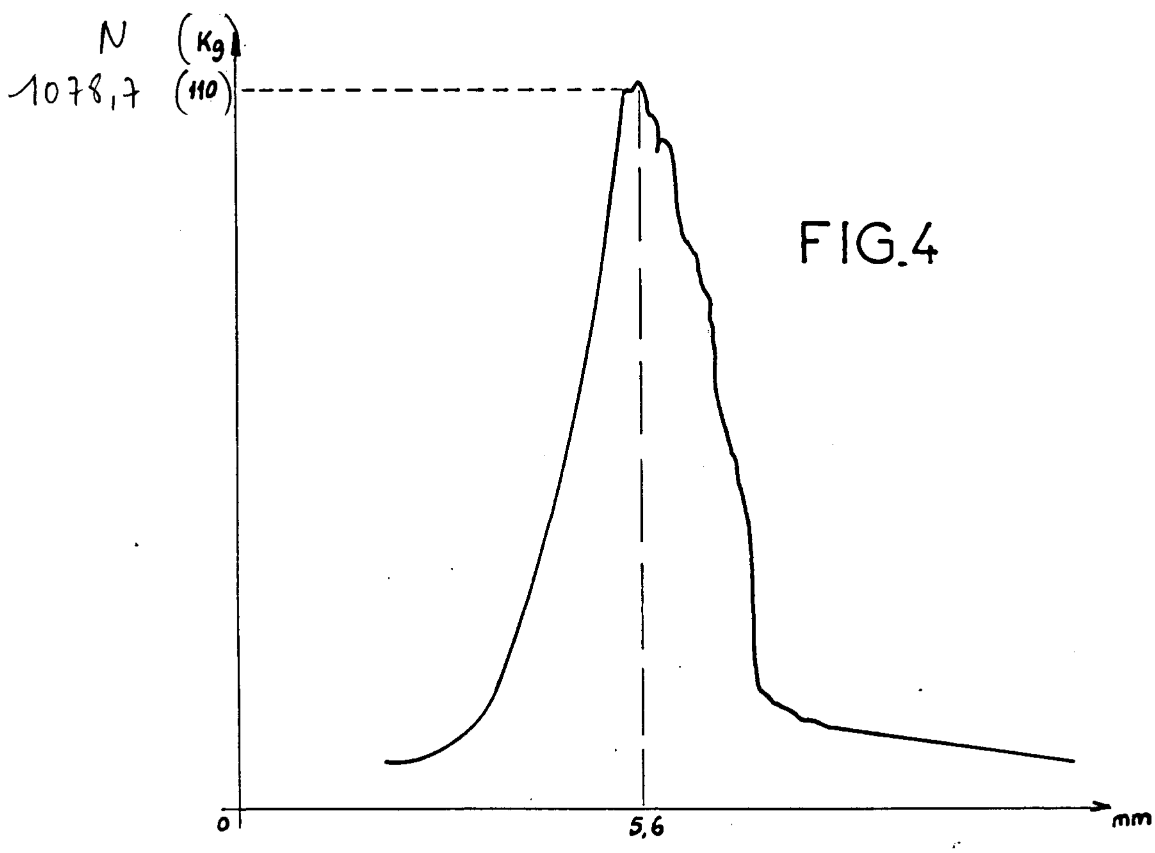


FIG.4

FIG. 5

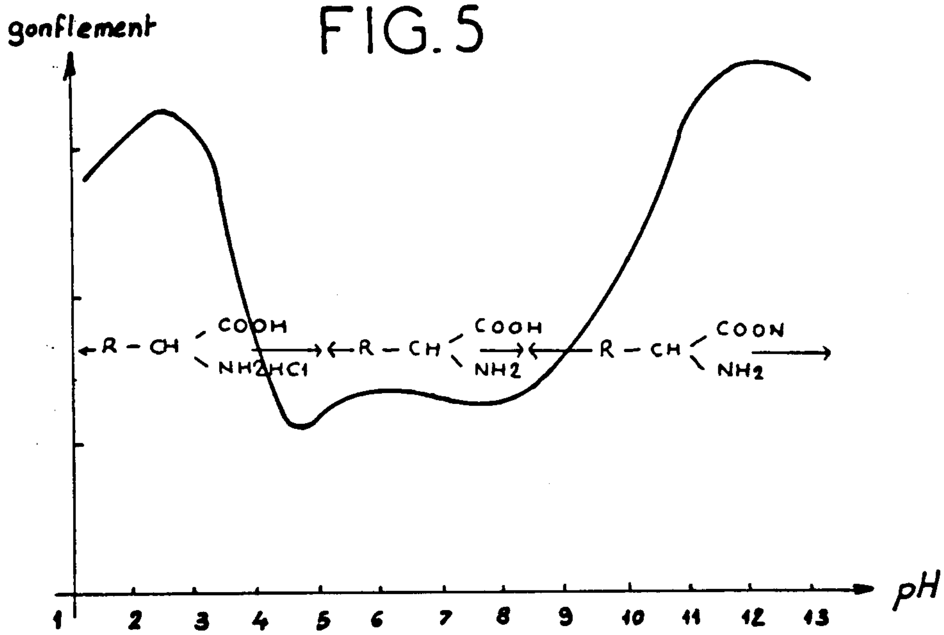
gonflement
R – CH COOH NH2HCl
R – CH COOH NH2
R – CH COON NH2
1
2
3
4
5
6
7
8
9
10
11
12
13
pH